# EUROPEAN PATENT APPLICATION

(11) **EP 3 109 820 A1**
(43) Date of publication of application: **28.12.2016**
(21) Application number: 15752823.3
(22) Date of filing: 16.02.2015
(51) Int. Cl.: G06Q 50/22

(54) **PROGRAM-IMPLEMENTATION ASSISTANCE DEVICE, PROGRAM-IMPLEMENTATION ASSISTANCE METHOD, AND RECORDING MEDIUM**

(30) Priority: 19.02.2014 JP 2014029710
(71) Applicant: NEC Solution Innovators, Ltd., Tokyo 136-8627 (JP)
(72) Inventor: YAMAMOTO, Kosuke, Tokyo 136-8627 (JP); OKAJIMA, Isa, Kodaira-shi Tokyo 187-0013 (JP)
(74) Representative: Betten & Resch
(86) International application number: PCT/JP2015/000694
(87) International publication number: WO 2015/125454

(57) **Abstract**

The present invention increases the implementation rate of an assigned lifestyle improvement program and increases implementation continuability. This program-implementation assistance device is equipped with: a task-associations-table storage means for storing a task-associations table in which the identification information for a task to be implemented as part of a prescribed lifestyle improvement program and identification information for a failure reason are associated with at least the identification information for another task to be implemented as part of the same lifestyle improvement program and accompanying a specific action corresponding to said failure reason; a reason acquisition means for making a user select a corresponding failure reason when implementation of the assigned task is insufficient; and a new-task selection means for selecting a new task to be implemented as part of the same lifestyle improvement program as the assigned task, on the basis of the assigned task identification information, the identification information for the failure reason selected by the user, and the task-associations table.

## Description

### Technical Field

The present invention relates to a measure implementation supporting apparatus, a measure implementation supporting method, and a recording medium for supporting the implementation of a predetermined lifestyle improvement measure.

### Background Art

Cognitive behavioral therapy is a methodology for changing thinking patterns of a person who tends to concentrate on bad ideas for a more balanced way of thinking by focusing on facts and behaviors. It has been reported that in the field of insomnia treatment, a therapeutic effect with a high remission rate can be obtained by an intervention of cognitive behavioral therapy. Cognitive behavioral therapy for insomnia is referred to as CBT-I (Cognitive-Behavioral Therapy for Insomnia), and CBT-I tools based on the concept of CBT-I such as Web services and self-care applications exist.

Generally, in CBT-I, coaching related to sleep hygiene, coaching related to sleep schedule, and coaching related to relaxation are provided.

In the coaching related to sleep hygiene, for example, habits and environments that may lead to awakening are learnt, and if there are such habits and environments, lifestyle improvement measures to improve such habits and environments are carried out. Furthermore, in the coaching related to sleep schedule, the importance of associating the bed or futon with a place to sleep is learnt, and lifestyle improvement measures for such association are carried out. In the coaching related to relaxation, the way of thinking and behavior for relaxing unnecessary force and preparation for making a sleeping state are learnt, and lifestyle improvement measures incorporating his/her own way of relaxing are carried out.

For example, the lifestyle improvement measures related to sleep hygiene include:
- Eat three substantial meals
- Do aerobic exercise such as walking
- Avoid afternoon nap
- Be in the sun for 30 minutes or longer within two hours after waking up in the morning
- Avoid caffeine consumption for six hours before going to bed
- Avoid smoking for one hour before going to bed
- Avoid alcohol consumption for four hours before going to bed
- Finish taking a bath by two hours before going to bed
- Avoid watching a screen with power supply such as television apparatus and personal computer for one hour before going to bed, etc.

The lifestyle improvement measures related to sleep schedule include:
- Restrict the time to be in bed from ○○ o'clock to ○○ o'clock (sleep restriction)
- Stay in futon or bed only while actually sleeping (sleep restriction)
- Get out of bed if one cannot fall asleep (stimulation control)
- Do not sleep in places other than bed.

The lifestyle improvement measurements related to relaxation include:
- Read for a short time before going to sleep
- Listen to music for a short time before going to sleep
- Bathe in a lukewarm bath
- Smell favorite fragrance before going to sleep
- Do muscle relaxing training.

In addition to the above-described coaching, psychoeducation such as analyzing the situation of a patient to give a correct knowledge on factors associated with his/her sleep, and if he/she has a habit in cognition or behavior that may prolong or worsen insomnia, to give advice or disclose data for correcting such cognition and habit may be carried out.

In CBT-I, the patient is given a daily assignment selected from unaccomplished assignments of the lifestyle improvement measures described above, thus removing habits detrimental to the sleep of the patient through the implementation of such an assignment. It is, temporarily, hard for the patient to carry out the assignment every day, but the effect can be expected to continue once the correct habits have been established.

As a technique utilizing the concept of CBT-I, for example, PTL 1 describes a system that obtains a correlation between a collected objective indices and a collected subjective indices related to sleep, and makes a proposal for improving sleep based on the result.

PTL 2 describes a system that gives warning, guidance, advice, or message encouraging a user, or proposes update of behavior to assist the user in complying with any selected behavior program.

### Citation List

### Patent Literature

- PTL 1:: WO 2008/096307
- PTL 2:: Japanese Patent No. 5307084

### Summary of Invention

### Technical Problem

It is known that CBT-I is more effective when a patient accomplishes his or her daily assignments given with a greater continuity, but in real situations it is difficult to have patients continuously accomplish their assignments.

One of the reasons patients fail to accomplish their assignments is that assignments tend to be not concrete enough. For example, if a lifestyle improvement measure "be in the sun for 30 minutes or longer within two hours after waking up in the morning" is given as an assignment without defining a specific behavior, quite a lot of patients fail to accomplish such an assignment.

To determine beforehand a specific behavior for every lifestyle improvement measure to be implemented, however, would require a great amount of time and labor, and hence is not realistic.

PTL 2 describes a devisal for encouraging the patient to implement the behavior program, that is, to propose to the patient that his or her behavior be updated, as, but what behavior the current behavior should be updated into is not disclosed.

It is thus an object of the present invention to provide a measure implementation supporting apparatus, a measure implementation supporting method, and a recording medium storing a measure implementation supporting program that can increase the implementation rate and the continuity of implementation of the lifestyle improvement measure assigned to a patient.

### Solution to Problem

A measure implementation supporting apparatus according to the present invention includes assignment association table storage means that stores an assignment association table in which identification information of an assignment for implementing a predetermined lifestyle improvement measure and identification information of a reason for failure defined in advance for the assignment are at least associated with identification information of another assignment for implementing the same lifestyle improvement measure, the another assignment involving a specific behavior in response to the reason for failure; reason acquisition means that, when implementation of a designated assignment is insufficient, allows a user to select a relevant reason for failure from reasons for failure defined in advance with respect to the assignment; and a new assignment selection means that selects a new assignment for implementing the same lifestyle improvement measure as the designated assignment, based on the identification information of the designated assignment, the identification information of the reason for failure selected by the user, and the assignment association table.

A measure implementation supporting method according to the present invention includes storing, in advance, in a predetermined storage means an assignment association table in which identification information of an assignment for implementing a predetermined lifestyle improvement measure and identification information of a reason for failure defined in advance for the assignment are at least associated with identification information of another assignment for implementing the same lifestyle improvement measure, the another assignment involving a specific behavior in response to the reason for failure; when implementation of a designated assignment is insufficient, allowing a user to select a relevant reason for failure from reasons for failure defined in advance with respect to the assignment; and selecting a new assignment for implementing the same lifestyle improvement measure as the designated assignment, based on the identification information of the designated assignment, the identification information of the reason for failure selected by the user, and the assignment association table.

A computer program to be installed in a computer to achieve the apparatus or the method described above, and a computer readable storage medium storing such a computer program are also included in the scope of the present invention.

### Advantageous Effects of Invention

According to the present invention, the implementation rate and the continuity of the implementation of a lifestyle improvement measure assigned to a patient can be increased.

### Brief Description of Drawings

[Fig. 1] Fig. 1 is a block diagram illustrating a configuration example of a measure implementation supporting apparatus according to a first exemplary embodiment.
[Fig. 2] Fig. 2 is an explanatory drawing illustrating an example of a highest order assignment.
[Fig. 3] Fig. 3 is a block diagram illustrating a configuration example of a new assignment recommendation unit.
[Fig. 4] Fig. 4 is a flowchart illustrating an operation example of the measure implementation supporting apparatus of the first exemplary embodiment.
[Fig. 5] Fig. 5 is an explanatory drawing illustrating an example of an assignment table stored in an assignment DB.
[Fig. 6] Fig. 6 is an explanatory drawing illustrating an example of a reason table stored in the assignment DB.
[Fig. 7] Fig. 7 is an explanatory drawing illustrating an example of an assignment association table stored in the assignment DB.
[Fig. 8A] Fig. 8A is an explanatory drawing illustrating an example of a user interface.
[Fig. 8B] Fig. 8B is an explanatory drawing illustrating an example of the user interface.
[Fig. 8C] Fig. 8C is an explanatory drawing illustrating an example of the user interface.
[Fig. 9A] Fig. 9A is an explanatory drawing illustrating another example of a new assignment.
[Fig. 9B] Fig. 9B is an explanatory drawing illustrating another example of a new assignment.
[Fig. 10] Fig. 10 is a block diagram illustrating a configuration example of a measure implementation supporting apparatus of a second exemplary embodiment.
[Fig. 11] Fig. 11 is a block diagram illustrating a configuration example of a new assignment recommendation unit.
[Fig. 12] Fig. 12 is an explanatory drawing illustrating an example of a reason table stored in an assignment DB.
[Fig. 13] Fig. 13 is an explanatory drawing illustrating an example of an assignment association table stored in the assignment DB.
[Fig. 14] Fig. 14 is a flowchart illustrating an operation example of the measure implementation supporting apparatus of the second exemplary embodiment.
[Fig. 15] Fig. 15 is an explanatory drawing illustrating an example of a user interface.
[Fig. 16] Fig. 16 is an explanatory drawing illustrating an example of the user interface.
[Fig. 17] Fig. 17 is an explanatory drawing illustrating an example of information stored in an implementation status DB.
[Fig. 18] Fig. 18 is an explanatory drawing illustrating an example of the user interface.
[Fig. 19] Fig. 19 is an explanatory drawing illustrating an example of the user interface.
[Fig. 20] Fig. 20 is an explanatory drawing illustrating an example of the user interface.
[Fig. 21] Fig. 21 is an explanatory drawing illustrating an example of the user interface;
[Fig. 22] Fig. 22 is a block diagram illustrating an outline of the present invention.
[Fig. 23] Fig. 23 is a view illustratively describing hardware configuration that can realize each exemplary embodiment.

### Description of Embodiments

### Exemplary Embodiment 1

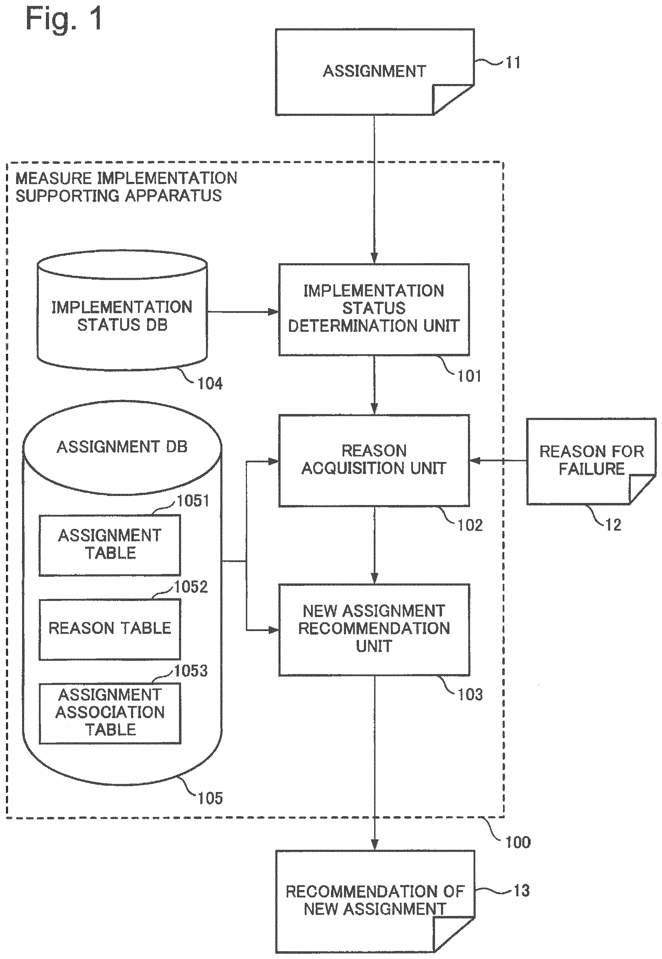

Fig. 1 is a block diagram illustrating a configuration example of a measure implementation supporting apparatus according to a first exemplary embodiment of the present invention. A measure implementation supporting apparatus 100 illustrated in Fig. 1 includes an implementation status determination unit 101, a reason acquisition unit 102, a new assignment recommendation unit 103, an implementation status database (implementation status DB (database)) 104, and an assignment database (assignment DB) 105.

The implementation status DB 104 stores information indicating an implementation status of an assignment currently given to a patient. The implementation status DB 104 stores, for example, information in which a date, an assignment ID (identifier) for identifying an assignment, and implemented or not implemented are associated with each other. In the present invention, a lifestyle improvement measure itself that is assigned to the patient, or a first assignment to the patient for implementing the lifestyle improvement measure are referred to as a highest order assignment related to the lifestyle improvement measure. In the present exemplary embodiment, with respect to a highest order assignment, one or more lower order assignments involving a specific behavior for implementing the same lifestyle improvement measure are defined in advance.

Upon receiving information 11 designating an assignment, the implementation status determination unit 101 determines the implementation status of the designated assignment. The implementation status determination unit 101 may determine whether the implementation of the designated assignment is sufficient, based on, for example, the information indicating the implementation status of the designated assignment stored in the implementation status DB 104. The implementation status determination unit 101 may determine whether the implementation of the assignment is sufficient according to, for example, a percentage and the number of non-implementations of the assignment indicated by the information indicating the implementation status of the designated assignment stored in the implementation status DB 104. Furthermore, when a plurality of assignments are designated, the implementation status determination unit 101 may determine the implementation status of each assignment, and output the assignment ID of the assignment of which the assignment implementation status is determined to be insufficient.

The assignment DB 105 stores an assignment table 1051, a reason table 1052, and an assignment association table 1053.

The assignment table 1051 is an information table holding the information of assignments in association with the assignment IDs of the assignments handled by the measure implementation supporting apparatus 100 of the present exemplary embodiment.

The reason table 1052 is an information table holding the information of failure reasons in association with reason IDs that identify reasons assumed for failure to accomplish assignments handled by the measure implementation supporting apparatus 100 of the present exemplary embodiment.

The assignment association table 1053 is an information table holding the relationship between assignments. More specifically, the assignment association table 1053 is an information table in which a first assignment, a second assignment which is another assignment involving a specific behavior for implementing the same lifestyle improvement measure as in the case of the first assignment, and a failure reason with respect to which the second assignment is introduced are associated with each other. The assignment association table 1053 may be, for example, information in which the information of the second assignment is associated with the assignment ID of the first assignment and the reason ID of a failure reason of the first assignment.

Hereinafter, a second assignment that defines a more specific behavior than the first assignment associated with the second assignment is sometimes referred to as an "in-depth assignment". An in-depth assignment is a second assignment in a subordinate relation to the associated first assignment. A second assignment that defines a specific behavior different from the first assignment associated with the second assignment is sometimes referred to as an "additional assignment". An additional assignment is a second assignment which is in a parallel relation to the associated first assignment. An additional assignment can also be regarded as a second assignment which is in a subordinate relation to an assignment superordinate to the first assignment with which the second assignment is associated.

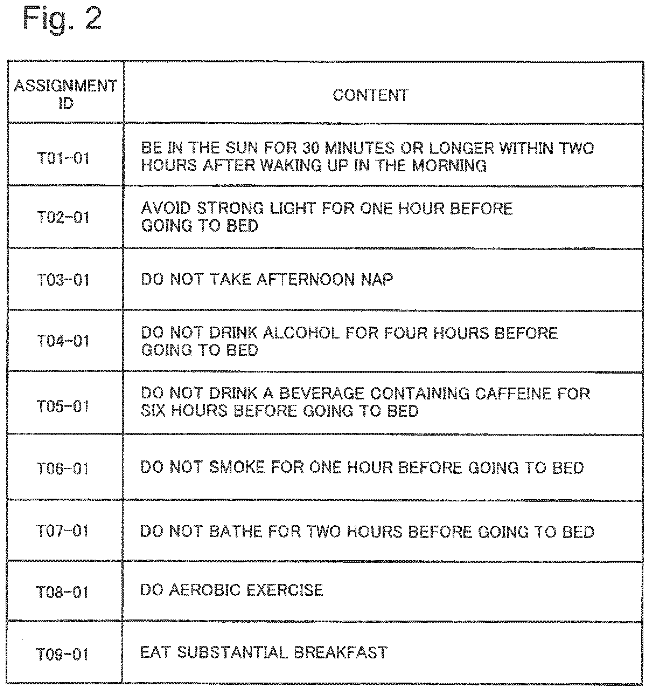

Fig. 2 is an explanatory drawing illustrating an example of the highest order assignments. In the present exemplary embodiment, a two digit-number following T of the each assignment ID represents the number for identification of the relevant lifestyle improvement measure, and a two-digit number following a hyphen represents the number for identification of each assignment in the assignments group for implementing the lifestyle improvement measure. The assignment table 1051, the reason table 1052, and the assignment association table 1053 may be provided for each lifestyle improvement measure.

The reason acquisition unit 102 asks a user a reason for failure of an assignment the implementation of which is determined to be insufficient (hereinafter referred to as a non-accomplished assignment), and acquires the reason for failure. The reason acquisition unit 102 refers to, for example, the assignment association table 1053, and by using the assignment ID of the non-accomplished assignment as the assignment ID of a first assignment, acquires a group of reason IDs associated with the assignment ID. The reason acquisition unit 102 then asks the user to select a reason for failure among the acquired group of reason IDs, presented as options, and acquires the reason for failure of the non-accomplished assignment (more specifically, information 12 indicating the reason for failure of the non-accomplished assignment). The information 12 indicating the failure reason is, for example, a reason ID.

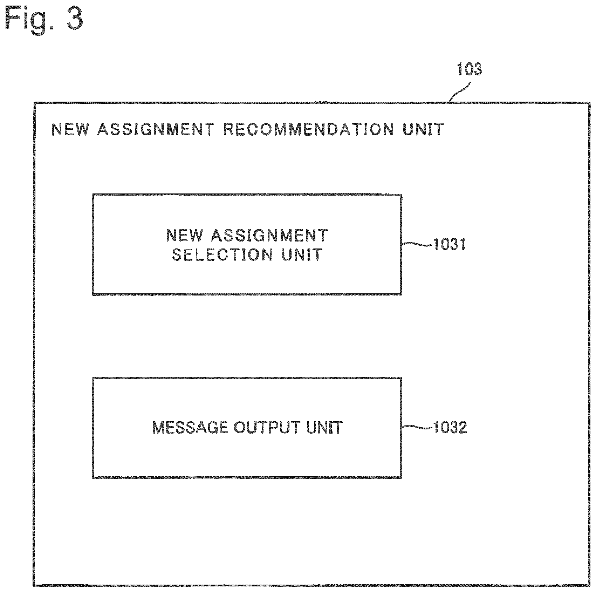

Fig. 3 is a block diagram illustrating a more detailed configuration example of the new assignment recommendation unit 103. As illustrated in Fig. 3, the new assignment recommendation unit 103 may include a new assignment selection unit 1031 and a message output unit 1032.

The new assignment selection unit 1031 selects a new assignment according to the reason for failure of the non-accomplished assignment selected by the user. The new assignment selection unit 1031 acquires information of a second assignment associated with the assignment ID of the non-accomplished assignment and the reason ID of the acquired reason for failure from, for example, the assignment association table 1053. The new assignment selection unit 1031 may select at least one of the assignments specified by the acquired information of the second assignment as a new assignment in response to the reason for failure of the non-accomplished assignment.

The message output unit 1032 outputs a message 13 recommending a new assignment to the user. The message output unit 1032 may output, as a recommendation message 13 of the new assignment, the content of the new assignment selected by the new assignment selection unit 1031 as well as, for example, a message recommending that the new assignment be set.

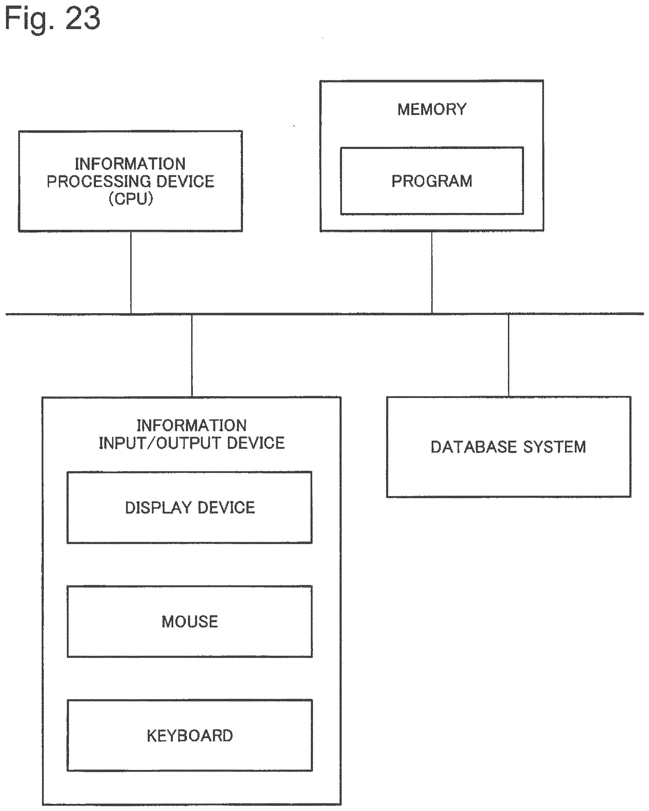

In the present exemplary embodiment, the implementation status determination unit 101, the reason acquisition unit 102, and the new assignment recommendation unit 103 (new assignment selection unit 1031, message output unit 1032) may, for example, be realized by hardware illustrated in Fig. 23. Specifically, as illustrated in Fig. 23, each unit described above is realized by an information processing device such as a CPU (Central Processing Unit) that operates in accordance with a program, and an information input/output device such as a display device, a mouse and a keyboard. The implementation status DB 104 and the assignment DB 105 are realized by, for example, storage means such as a memory or a database system, as illustrated in Fig. 23. A recording medium that stores a program readable by the CPU may be a nonvolatile recording medium.

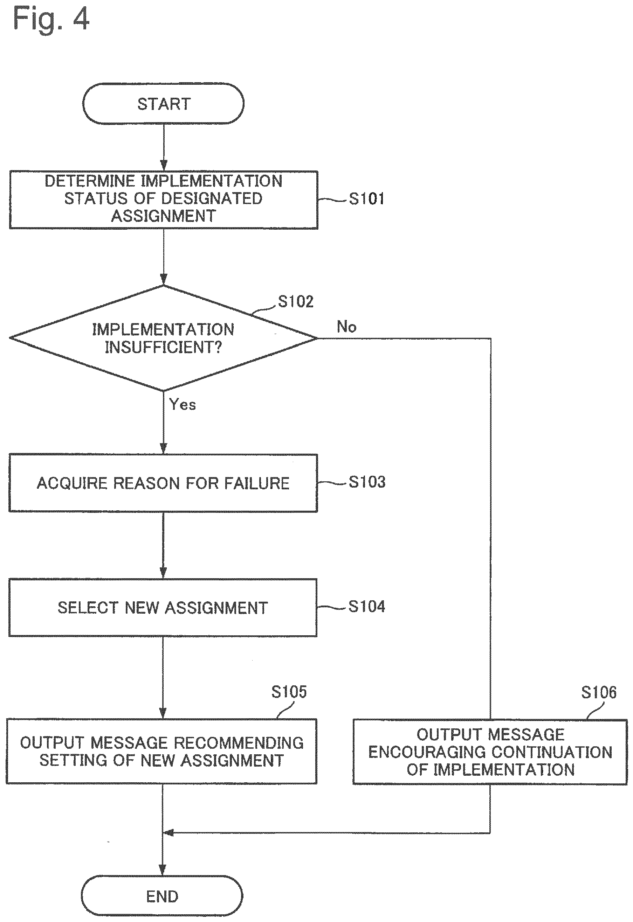

The operation of the present exemplary embodiment will now be described. Fig. 4 is a flowchart illustrating an example of the operation of the present exemplary embodiment. As illustrated in Fig. 4, when the information 11 designating an assignment is input, the implementation status determination unit 101 first determines the implementation status of the designated assignment (step S101). When it is determined as a result that the implementation is insufficient (Yes in step S102), the reason acquisition unit 102 acquires a reason for failure of the designated assignment from the user (step S103).

The new assignment selection unit 1031 then selects a new assignment according to the acquired reason for failure (step S104).

After the new assignment is selected, the message output unit 1032 outputs the recommendation message 13 of the selected new assignment (step S105). The recommended new assignment is added, for example, with consent by the user, to the assignments for implementing the same lifestyle improvement measure which the non-accomplished assignment concerns.

In the example described above, the new assignment is added as one of the assignments for implementing the lifestyle improvement measure so that the user is not restricted by the specific behavior defined by the new assignment and does not feel uncomfortable. The new assignment selection unit 1031 may add the presented new assignment to the assignments to be given to the user without consent of the user. In such a case, the process of step S105 is omitted or is replaced with a process of outputting a report message of the set assignment.

When, on the other hand, it is determined that the implementation is sufficient (No in step S102), the message output unit 1032 may output a message encouraging the continuation of the implementation of the assignment (step S106).

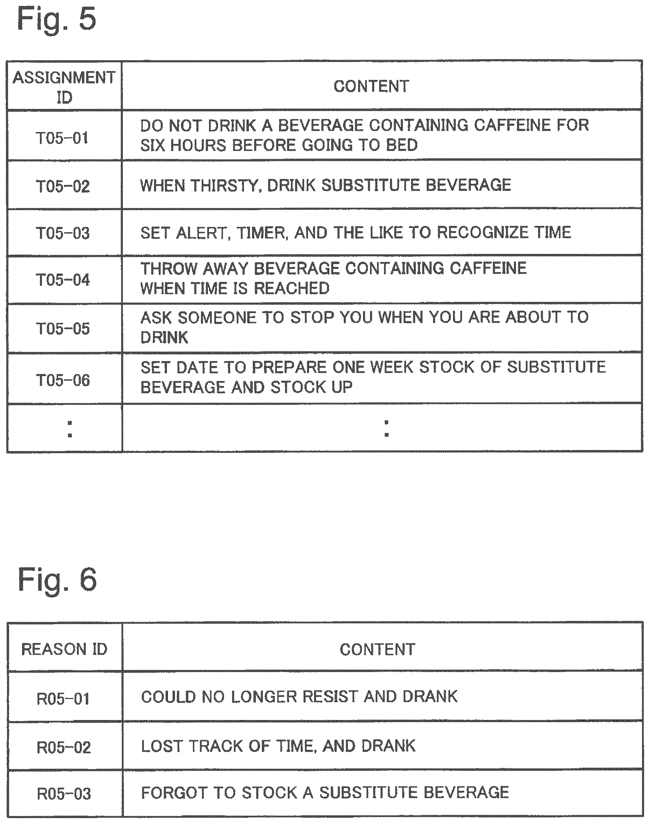

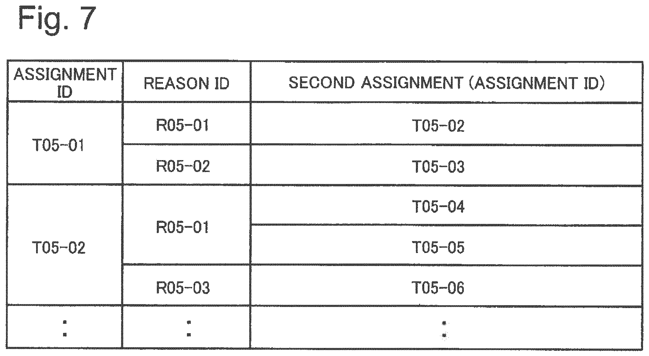

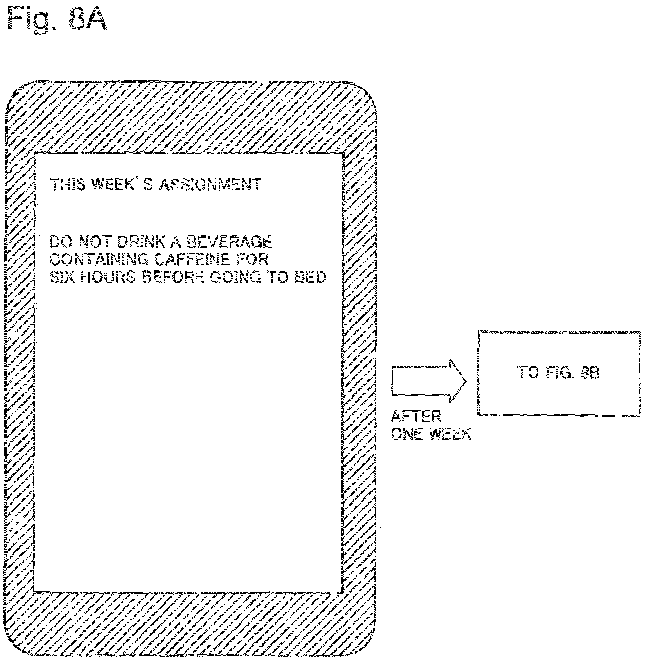

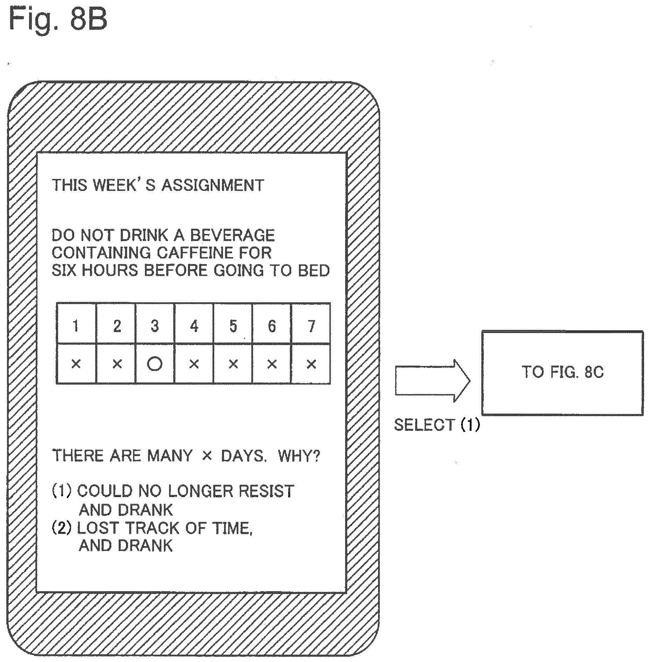

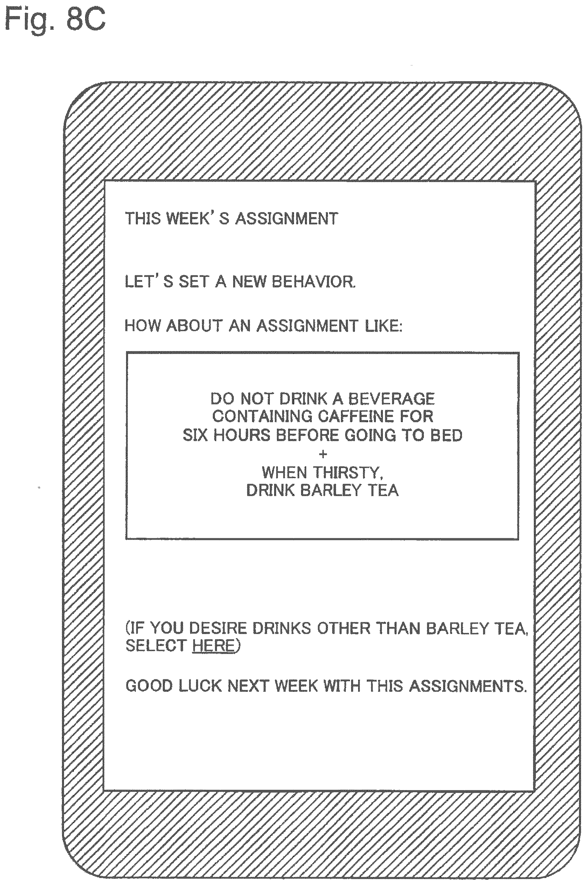

Next, the operation of the present exemplary embodiment will be described using a specific example. Fig. 5 is an explanatory drawing illustrating an example of the assignment table 1051 stored in the assignment DB 105. Fig. 6 is an explanatory drawing illustrating an example of the reason table 1052 stored in the assignment DB 105. Fig. 7 is an explanatory drawing illustrating an example of the assignment association table 1053 stored in the assignment DB 105. Figs. 8A, 8B, and 8C are explanatory drawings illustrating an example of a user interface in the present example.

As an example of the assignment table 1051, Fig. 5 illustrates a part of the information table holding the assignment ID of each assignment included in an assignment group having one of the lifestyle improvement measures assigned to the patient based on cognitive behavioral therapy for insomnia in the highest order , more specifically, an assignment group including a highest order assignment and lower order assignments derived therefrom, and the content of the assignment in association with each other. The assignment ID = T05-01 is the assignment ID of the lifestyle improvement measure, which is the highest order assignment, of the present example.

As an example of the reason table 1052, Fig. 6 illustrates a part of the information table holding the reason ID of each reason for failure included in a group of reasons for failure assumed for one of the lifestyle improvement measures assigned to the patient based on cognitive behavioral therapy for insomnia, and the content of the reason for failure in association with the reason ID. In the present example, the two-digit number following R of the reason ID represents the number for identification of the lifestyle improvement measure, which is the highest order assignment, and the two-digit number following a hyphen represents the number for identification of each reason for failure in the group of reasons for failure.

As an example of the assignment association table 1053, Fig. 7 illustrates a part of the information table holding the assignment ID of each assignment included in the assignment group having a highest order assignment with the assignment ID = T0S-01, the reason IDs of the reasons for failure assumed for the relevant assignment, and the assignment IDs of the second assignments in response to the relevant reasons for failure, associated with each other.

As illustrated in Fig. 8A, assume that the assignment "do not drink a beverage containing caffeine for six hours before going to bed" with the assignment ID = T05-01 is assigned to the patient.

In the present example, first, the assignment ID = {T05-01} of the current assignment is input to the implementation status determination unit 101 as the information 11 designating the assignment. In this case, as illustrated in Fig. 8B, suppose the implementation rate of the relevant assignment during a predetermined period is 1/7. The implementation status determination unit 101 refers the information indicating the implementation status of the relevant assignment from the implementation status DB 104, and determines that the implementation of the relevant assignment is insufficient.

For example, when an assignment ID is input as information 11 designating an assignment, the implementation status determination unit 101 may acquire a record indicating the implementation status of the assignment of the assignment ID that matches the input assignment ID from the implementation status DB 104 for a predetermined period, and calculate the number of the assignment implementation during the period based on the acquired group of records. The implementation status determination unit 101 may determine that the implementation is insufficient when the number of the implementation is lower than a predetermined threshold value.

When the implementation status determination unit 101 determines that the implementation is insufficient, the reason acquisition unit 102 asks the user the reason for failure of the assignment (non-accomplished assignment). For example, the reason acquisition unit 102 obtains a reason ID = {R05-01, R05-02} associated with the assignment ID = {T05-01} of the non-accomplished assignment from the assignment association table 1053 illustrated in Fig. 7. The reason acquisition unit 102 then refers the reason table 1052 illustrated in Fig. 6, using the acquired reason ID, and obtains a reason for failure "could no longer resist and drank" with the reason ID = R05-01 and a reason for failure "lost track of time, and drank" with the reason ID = R05-02. The reason acquisition unit 102 then outputs a message for asking the user to select a reason for failure from the reasons for failure obtained as options (see Fig. 8B).

Suppose the user selects the reason for failure "could no longer resist and drank" with the reason ID = R0S-01. As a result of the selection, the reason acquisition unit 102 obtains the reason ID = R05-01 as information 12 indicating the reason for failure.

The new assignment selection unit 1031 selects a new assignment in response to the reason for failure acquired by the reason acquisition unit 102. In the present example, the new assignment selection unit 1031 obtains from the assignment association table 1053 illustrated in Fig. 7, the assignment ID = {T05-02} of a second assignment which is associated with the assignment ID = T05-01 of the non-accomplished assignment and with the reason ID = R05-01 acquired by the reason acquisition unit 102. The assignment indicated by the acquired assignment ID of the second assignment is selected as a new assignment.

After a new assignment is selected, the message output unit 1032 refers the assignment table 1051 illustrated in Fig. 5, using the assignment ID = {T05-02} of the new assignment selected by the new assignment selection unit 1031. The message output unit 1032 then outputs the content of the obtained new assignment, and the recommendation message 13 recommending that a setting of the obtained new assignment. As illustrated in Fig. 8C, in the present exemplary embodiment, a combination of the content of the current assignment and the content of the obtained new assignment, such as "do not drink a beverage containing caffeine for six hours before going to bed + when thirsty, drink substitute beverage" may be output as a new assignment.

Fig. 8C illustrates an example in which the "substitute beverage" included in the content of the obtained new assignment is specified as "barley tea" and subsequently presented to the user, but the substitute beverage may be specified by the user. When, for example, the content of the obtained new assignment includes a predetermined symbol or the like indicating an item to be designated by the user, the new assignment selection unit 1031 or the message output unit 1032 may provide the user with a user designated item input screen or the like for specifying the user designated item, and allow the user to input a specific item to which is to be set as the user designated item.

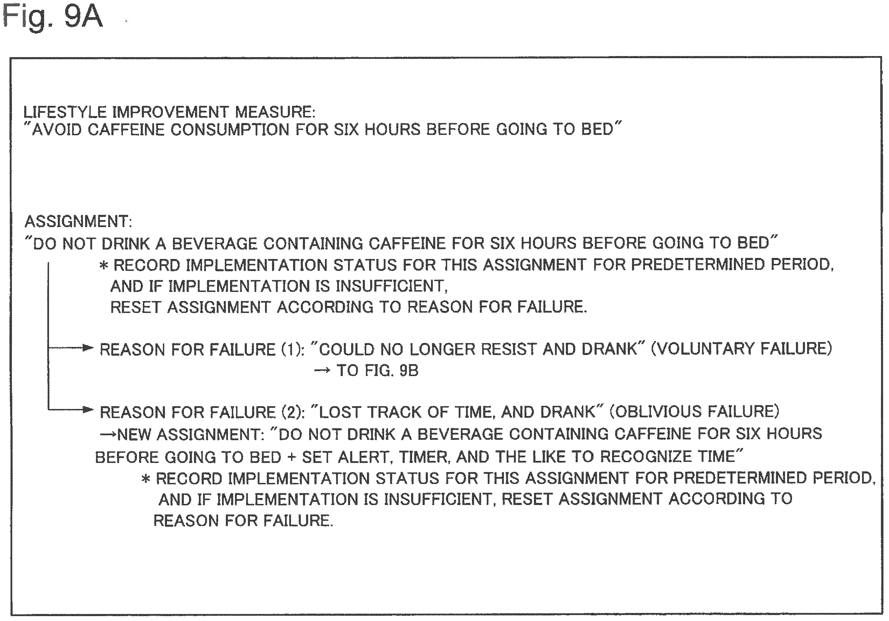

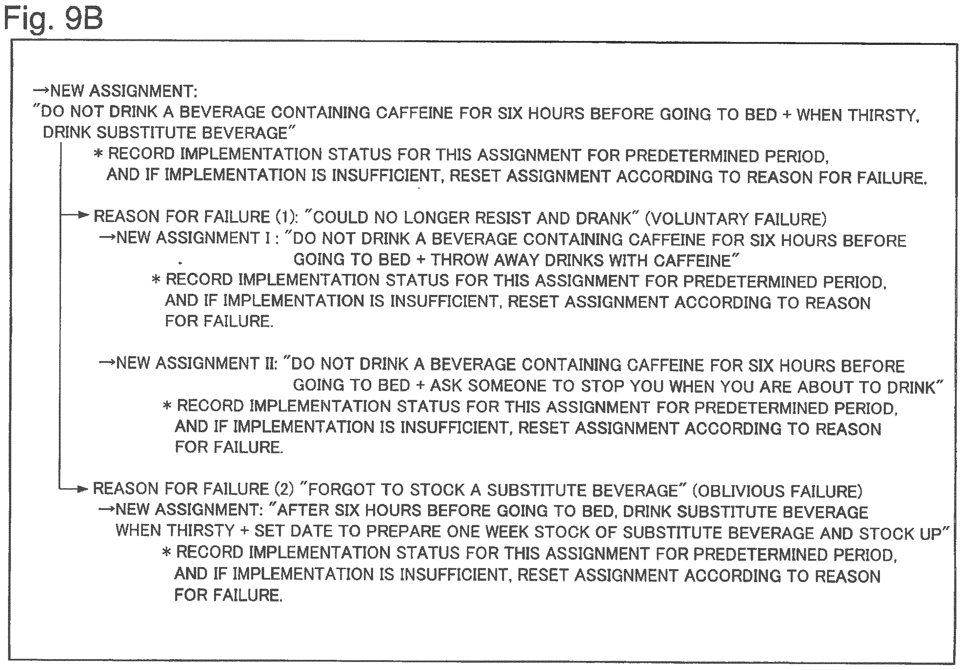

Figs. 9A and 9B are explanatory drawings illustrating another example of a new assignment. As illustrated in Fig. 9A, for example, a case in which the reason for failure "lost track of time, and drank" with the reason ID = R05-02 is selected for the reason for failure of the designated assignment (assignment ID = T05-01) will be described. In this case, "do not drink a beverage containing caffeine for six hours before going to bed + set alert, timer, or the like to recognize time" is selected and presented as a new assignment, based on the assignment ID = {T05-03} of the second assignment, which is associated with the assignment ID = T05-01 of the designated assignment and the reason ID = R05-02 of the selected reason for failure.

Furthermore, for example, suppose that another new assignment (e.g., assignment ID = T05-02) is set as one of the assignments for the subsequent period. Assume that, when the implementation status of the new assignment (assignment = ID = T05-02) is determined after a predetermined time, the implementation is determined to be insufficient. In such a case, the reason acquisition unit 102 acquires the reason for failure of the set new assignment (assignment ID = T05-02), the new assignment selection unit 1031 selects a new assignment in response to the acquired reason for failure, and the message output unit 1032 outputs the recommendation message 13 of the selected new assignment through a method similar to the above.

For example, with reference to the assignment association table 1053 illustrated in Fig. 7, the reason for failure "could no longer resist and drank" with the reason ID = R05-01 and the reason for failure "forgot to stock a 'substitute beverage'" with the reason ID = R05-03 are acquired as the reasons for failure assumed for the assignment with assignment ID = T05-02.

Here, a case in which the user selects the reason for failure "could no longer resist and drank" with the reason ID = R05-01, for example, will be described. In this case, the assignment with the assignment ID = T05-04 and the assignment with the assignment ID = T05-05 are selected, based on the assignment ID = T05-02 of the non-accomplished assignment and the reason ID = R05-01 of the reason for failure selected by the user. Thus, the contents "do not drink a beverage containing caffeine for six hours before going to bed + throw away drinks with caffeine when the time is reached" and "do not drink a beverage containing caffeine for six hours before going to bed + ask someone to stop you when you are about to drink" obtained from the two selected assignments are presented as the recommendation message 13.

A case in which the user selects the reason for failure "forgot to stock a substitute beverage" of the reason ID = R05-03, for example, will be described. In this case, the assignment "within six hours before going to bed, drink "a substitute beverage" when thirsty + set a date to prepare one week stock of 'a substitute beverage' and stock up" based on the assignment ID = T05-06 is selected as a new assignment, based on the assignment ID = T05-02 of the non-accomplished assignment and the reason ID = R05-01 of the reason for failure selected by the user. The content of the selected new assignment is presented by the recommendation message 13. Thus, an interaction is realized through which the contents of the assignment, the implementation of which turned out to be insufficient, can be changed step by step according to the reasons for failure.

As described above, the measure implementation supporting apparatus according to the present exemplary embodiment holds reasons for failure assumed for each assignment and second assignments for responding to the reasons, in association with each other. Upon obtaining information that shows a failure to accomplish the assignment, the apparatus allows the user to select a relevant reason for failure from the reasons for failure assumed for the assignment, and outputs a message recommending a new assignment involving a specific behavior in response to the reason for failure. Since the apparatus is provided with an interaction which easily allows to present in concrete terms a behavior for accomplishing the assignment which the user has failed to accomplish, the user can recognize in concrete terms a behavior for implementing the assignment and easily carry out the lifestyle improvement measure without much trouble. Therefore, the implementation rate of the assigned lifestyle improvement measure and the continuity of the implementation can be increased.

### Exemplary Embodiment 2

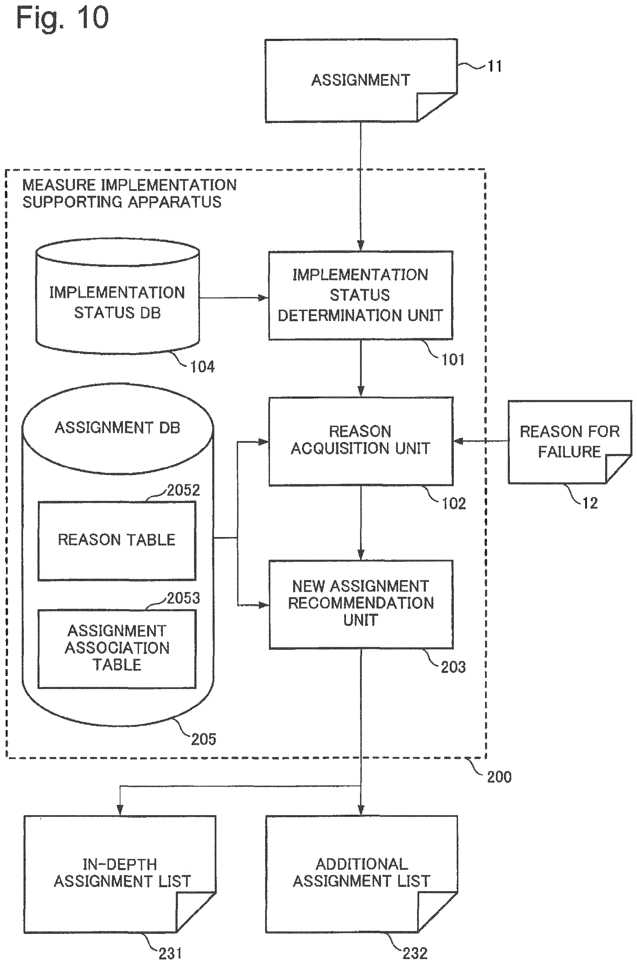

Fig. 10 is a block diagram illustrating a configuration example of a measure implementation supporting apparatus according to a second exemplary embodiment of the present invention. A measure implementation supporting apparatus 200 illustrated in Fig. 10 differs from the measure implementation supporting apparatus 100 according to the first exemplary embodiment illustrated in Fig. 1 in that the new assignment recommendation unit 103 is replaced with a new assignment recommendation unit 203, and the assignment DB 105 is replaced with an assignment DB 205.

The assignment DB 205 stores a reason table 2052 and an assignment association table 2053.

The reason table 2052 is an information table holding the information of reasons for failure assumed for each assignment handled by the measure implementation supporting apparatus 200, in association with reason IDs that identify the reasons for failure. The reason table 2052 of the present exemplary embodiment holds, as information on each reason for failure, information that indicates the type of new assignments that can be set when the reason for failure is the relevant one. The reason table 2052 may be, for example, an information table holding the reason IDs, the content of the reasons for failure, and an in-depth flag and an addition flag for the information indicating the type of another assignment that can be set in association with each other. An in-depth flag is a flag that represents whether an assignment that is regarded as an in-depth assignment can be set with respect to the assignment designated as a new assignment. An additional flag is a flag that represents whether an assignment that is regarded as an additional assignment can be set with respect to the assignment designated as a new assignment.

The assignment association table 2053 is an information table in which a function of the assignment association table 1053 and a function of the assignment table 1051 of the first exemplary embodiment are combined. In other words, the assignment association table 2053 is an information table holding the relationship between the assignments along with the information related to at least one assignment. The assignment association table 2053 may, for example, for each of the assignments handled by the measure implementation supporting apparatus 200 of the present exemplary embodiment, be information in which the assignment ID, a parent ID, which is an assignment ID of a parent assignment of the relevant assignment, the content of the relevant assignment, the reason for failure of the parent assignment in response to which the relevant assignment is given, and the information of the reasons for failure assumed for the relevant assignment are associated with each other.

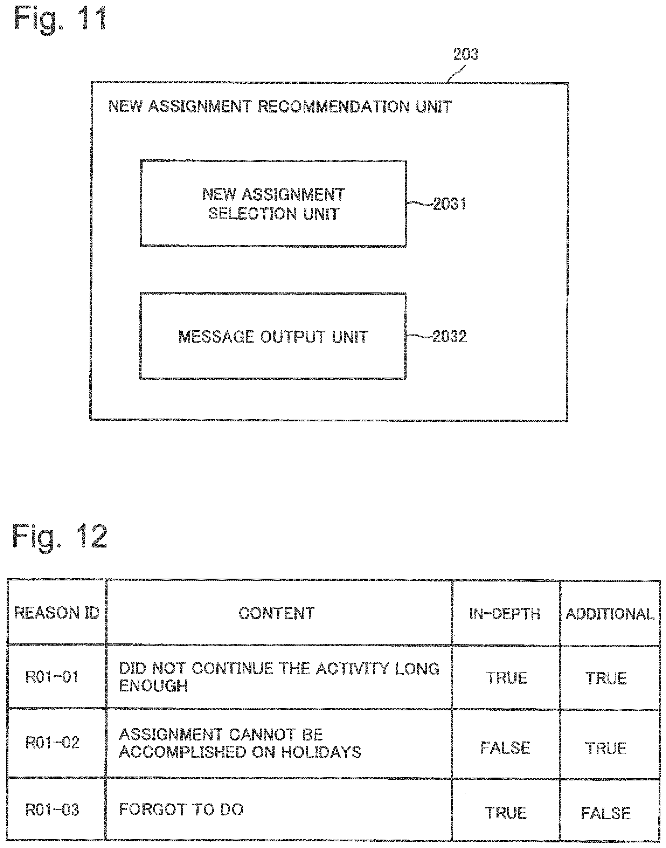

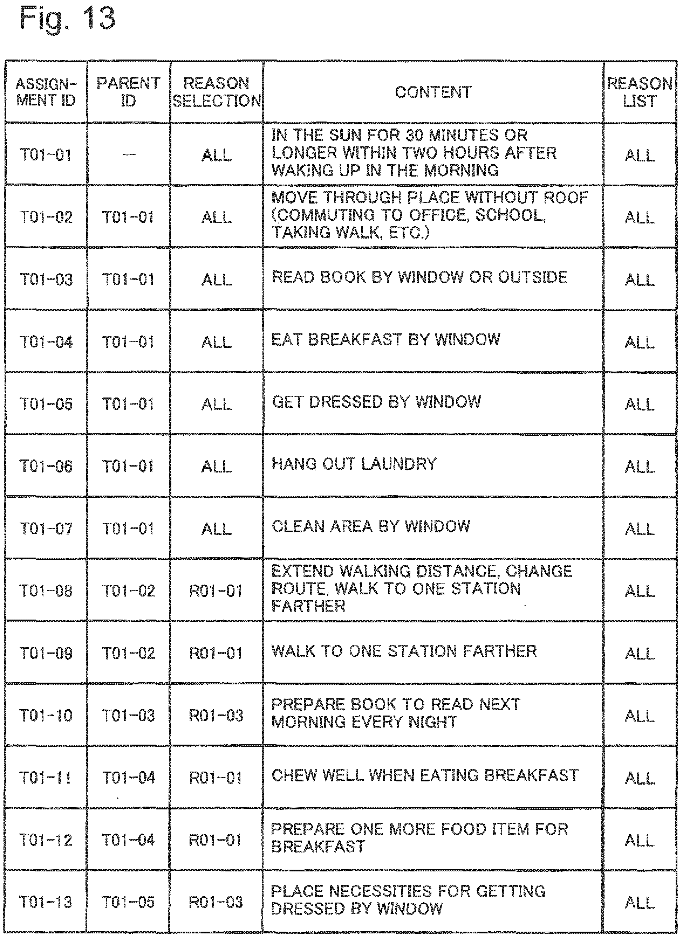

Fig. 12 is an explanatory drawing illustrating an example of the reason table 2052 stored in the assignment DB 205. Fig. 13 is an explanatory drawing illustrating an example of the assignment association table 2053 stored in the assignment DB 205.

As an example of the reason table 2052, Fig. 12 illustrates an information table holding the reason ID of each reason for failure included in the group of reasons for failure assumed for one of the lifestyle improvement measures assigned to the patient based on cognitive behavioral therapy for insomnia, the contents of the reasons for failure, and the in-depth flag and the additional flag presented as types of second assignments, in association with each other. In the present example, a two-digit number following R in a reason ID represents the number for identification of the lifestyle improvement measure, which is the highest order assignment of the present example, and a two-digit number following a hyphen represents the number for identification of each reason for failure in the group of reasons for failure.

As an example of the assignment association table 2053, Fig. 13 illustrates an information table holding the assignment ID of each assignment included in the assignment group having one of the lifestyle improvement measures assigned to the patient based on cognitive behavioral therapy for insomnia as the highest order assignment, parent IDs, reason selection, and a reason list in association with each other. Reason selection presents information of the reasons for failure of the parent assignment in response to which the assignment in question is given. The reason list is the information of the reasons for failure assumed for the relevant assignment. In the present example, a two-digit number following T of an assignment ID represents the number for identification of the lifestyle improvement measure, which is the highest order assignment, of the present example, and a two-digit number following a hyphen represents the number for identification of each assignment in the assignment group.

Fig. 11 is a block diagram illustrating a more detailed configuration example of the new assignment recommendation unit 203. As illustrated in Fig. 11, the new assignment recommendation unit 203 may include a new assignment selection unit 2031 and a message output unit 2032.

The new assignment selection unit 2031 selects a new assignment according to the reason for failure of the non-accomplished assignment selected by the user, similarly to the first exemplary embodiment. The new assignment selection unit 2031 of the present exemplary embodiment selects an in-depth assignment, which is an assignment subordinate to the non-accomplished assignment, and/or an additional assignment, which is an assignment in the equal order to the non-accomplished assignment, as a new assignment, based on the information indicating the propriety of second assignments by type, which is registered in advance, with respect to the reasons for failure acquired by the reason acquisition unit 102.

The message output unit 1032 outputs a recommendation message of a new assignment including an in-depth assignment list 231, which is a list of in-depth assignments, and/or a list of additional assignments based on the result of the selection by the new assignment selection unit 2031.

When, for example, the in-depth flag associated with the acquired reason for failure has a sign "TRUE", representing that the setting of an in-depth assignment is possible, the new assignment selection unit 2031 selects an assignment that meets the conditions from the assignments having the non-accomplished assignment as a parent in the assignment association table 2053 as an in-depth assignment serving as a new assignment (hereinafter referred to as new in-depth assignment). More specifically, the new assignment selection unit 2031 selects an assignment such that the assignment ID of the non-accomplished assignment is the parent ID and the acquired reason for failure is included in the reason selection as a new in-depth assignment as the in-depth assignment list 231. The message output unit 2032 may, for example, list up the selected new in-depth assignments to create an in-depth assignment list 231, and output a recommendation message recommending the setting of an assignment included in the created in-depth assignment list 231.

When, for example, the additional flag associated with the acquired reason for failure has a sign "TRUE", representing that the setting of an additional assignment is possible, the new assignment selection unit 2031 selects an assignment that meets the conditions from the assignments sharing a parent with the non-accomplished assignment in the assignment association table 2053 as an additional assignment serving as a new assignment (hereinafter referred to as new additional assignment). More specifically, the new assignment selection unit 2031 selects an assignment that has the same parent ID as the parent ID of the non-accomplished assignment such that it is not currently set as an assignment and that the acquired reason for failure is included in the reason selection as a new additional assignment. The message output unit 2032 may, for example, list up the selected new additional assignments to create an additional assignment list 232, and output a recommendation message recommending the setting of an assignment included in the additional assignment list 232.

The processing of creating the in-depth assignment list 231 and the additional assignment list 232 may be carried out by the new assignment selection unit 2031.

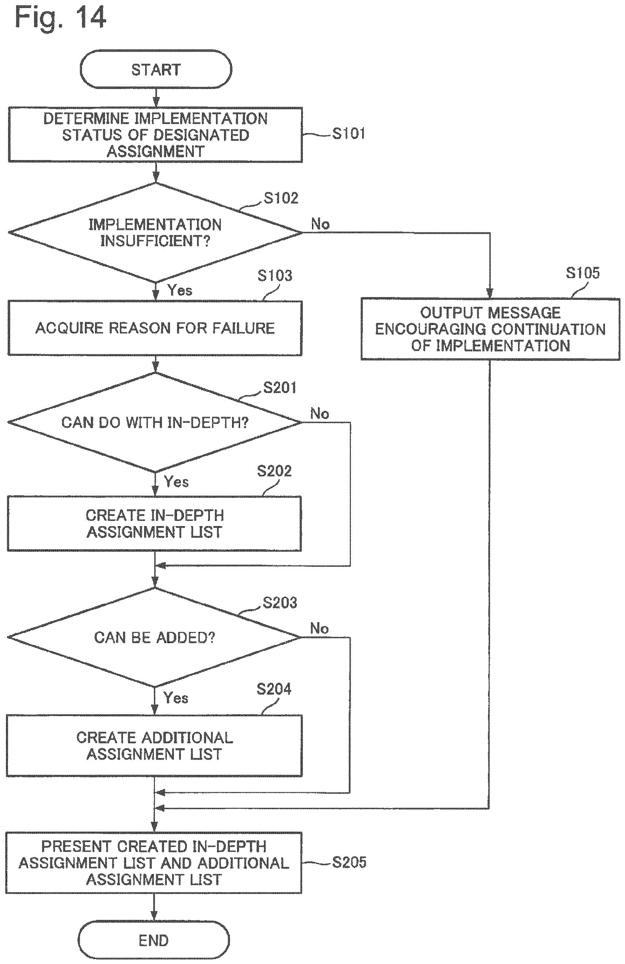

Next, the operation of the present exemplary embodiment will be described. Fig. 14 is a flowchart illustrating one example of the operation of the present exemplary embodiment. The steps same as in the first exemplary embodiment are denoted with the same step numbers, and the description thereof will be omitted.

In the present exemplary embodiment, when the reason acquisition unit 102 acquires a reason for failure of the non-accomplished assignment (step S103), the new assignment selection unit 2031 checks the in-depth flag and the additional flag associated with the acquired reason for failure.

When the in-depth flag associated with the acquired reason for failure represents that the setting of an in-depth assignment is possible (Yes in step S201), the new assignment selection unit 2031 selects a new in-depth assignment based on the assignment ID of the non-accomplished assignment, the acquired reason for failure, and the assignment association table 2053, and creates an in-depth assignment list 231 (step S202).

When the additional flag associated with the acquired reason for failure represents that the adding an additional assignment is possible (Yes in step S203), the new assignment selection unit 2031 selects a new additional assignment, based on the assignment ID of the non-accomplished assignment, the acquired reason for failure, and the assignment association table 2053, and creates an additional assignment list 232 (step S204).

The message output unit 2032 presents the created in-depth assignment list 231 and the created additional assignment list 232, and outputs a recommendation message recommending that an assignment included in the in-depth assignment list 231 be set as an in-depth assignment and a recommendation message recommending that an assignment included in the additional assignment list 232 be set as an additional assignment (step S205).

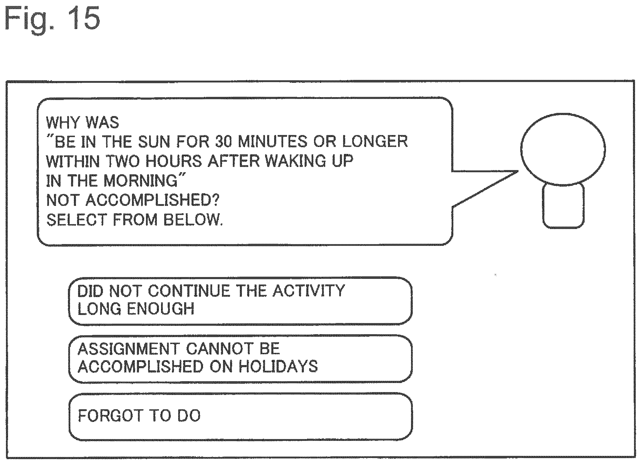

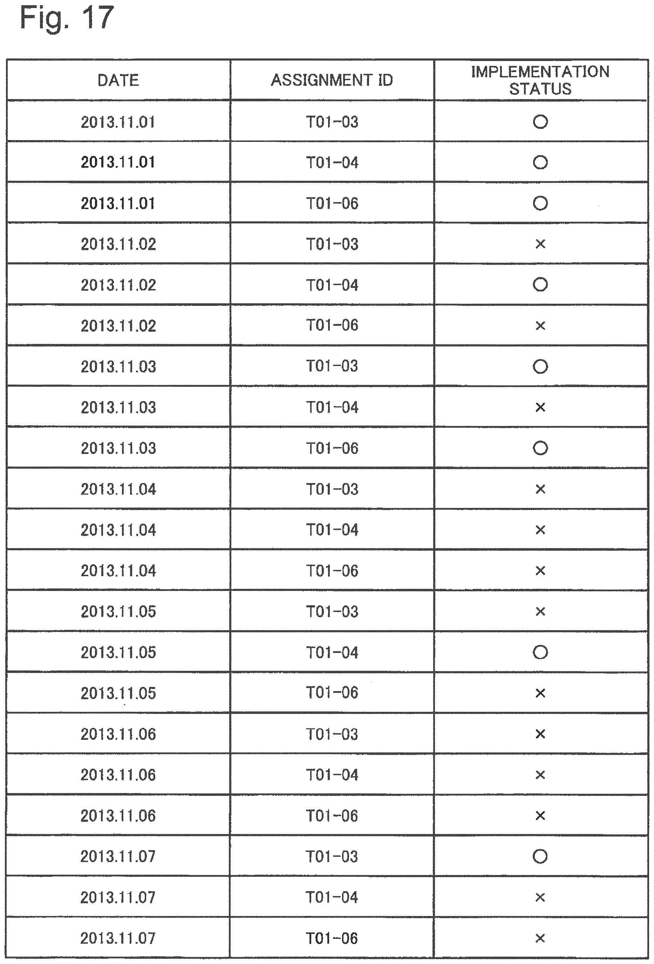

Next, the operation of the present exemplary embodiment will be described using a specific example. Figs. 15 and 16, as well as Figs. 18 to 21 are explanatory drawings illustrating an example of the user interface in the present example. Fig. 17 is an explanatory drawing illustrating an example of the information stored in the implementation status DB 104. Suppose that an assignment "Be in the sun for 30 minutes or longer within two hours after waking up in the morning" with the assignment ID = T01-01 is given to the patient.

In the present example, the assignment ID = {T01-01} of the current assignment is first input as the information 11 designating the assignment, to the implementation status determination unit 101. In this case, assume that the implementation status of the relevant assignment during a predetermined period is insufficient. The implementation status determination unit 101 refers the implementation status of the assignment in the implementation status DB 104, and determines that the implementation of the relevant assignment is insufficient.

When the implementation status determination unit 101 determines that the implementation is insufficient, the reason acquisition unit 102 asks the user the reason for failure regarding the relevant assignment (non-accomplished assignment). For example, the reason acquisition unit 102 acquires the reason list associated with the assignment ID = {T01-01} of the non-accomplished assignment from the assignment association table 2053 illustrated in Fig. 13. The reason acquisition unit 102 then asks the user to select a reason for failure from the acquired reason list presented as options. "ALL" in the reason list field of the assignment association table 2053 illustrated in Fig. 13 means that all the reasons included in the reason table 2052 are relevant. Therefore, the reason acquisition unit 102 inquires the reason for failure of the non-accomplished assignment by presenting to the user the reason ID = {R01-01, R01-02, R01-03} as options for a reason for failure to be selected (see Fig. 15).

Suppose here that the user selects the reason for failure "did not continue the activity long enough" with the reason ID = R01-01, for example. Upon receiving the selection result, the reason acquisition unit 102 obtains the reason ID = R01-01 as the information 12 indicating the reason for failure.

The new assignment selection unit 2031 acquires the assignment IDs having assignment ID = T01-01 as the parent ID, the assignment ID being the non-accomplished assignment, from the assignment association table 2053 since the value of the in-depth flag associated with the reason for failure (reason ID = R01-01) is "TRUE" in the reason table 2052. However, assignment IDs with the reason selection field thereof in the assignment association table 2053 not containing the reason for failure selected by the user are removed from the acquired assignment IDs, that is, the assignment IDs having the assignment ID = T01-01 as the parent ID thereof. The new assignment selection unit 2031 stores the ultimately remaining assignments in the in-depth assignment list 231. In the present example, the assignment IDs = {T01-02 to T01-07} are acquired since they are assignment IDs having assignment ID = T01-01 as the parent ID thereof. "ALL" in the reason selection field of the assignment association tale 2053 illustrated in Fig. 13 means that all the reasons included in the reason table 2052 are relevant. Therefore, the assignments with the acquired assignment IDs = {T01-02 to T01-07} are listed up to create an in-depth assignment list 231. The assignments with the assignment IDs = {T01-02 to T01-07} specify a behavior for implementing the parent assignment, i.e., the assignment with the assignment ID = {T01-01}.

The message output unit 2032 outputs a message recommending that more specific assignments be set and recommends assignments in the created in-depth assignment list 231.

The new assignment selection unit 2031 acquires the assignment ID sharing a parent ID with the non-accomplished assignment (assignment ID = T01-01) from the assignment association table 2053 since the value of the additional flag associated with the reason for failure (reason ID = R01-01) of the non-accomplished assignment is "TRUE" in the reason table 2052. However, the current assignment and the parent assignment thereof (recursively up to root) are excluded. The new assignment selection unit 2031 stores the ultimately remaining assignment in the additional assignment list 232. In the present example, the parent ID of the assignment ID = T01-01 is "- -", that is, not applicable, and hence the assignment to be added is not acquired, and the additional assignment list 232 is empty.

When at least one assignment is included in the created additional assignment list 232, the message output unit 2032 outputs a message recommending the addition of assignment and recommends assignments in the created additional assignment list 232 so that the user does not stick to one method.

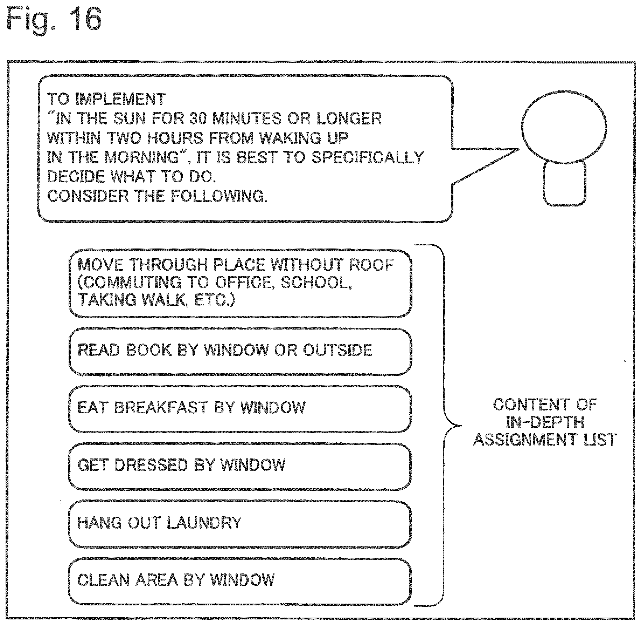

In the present example, the in-depth assignment list 231 including the assignments of assignment ID = {T01-02 to T01-07} is eventually presented with the message recommending the setting of the assignments included in the list (see Fig. 16).

When, for example, the user selects the assignment IDs = {T01-03, T01-04, T01-06}, the new assignment selection unit 2031 may set the selected assignments as assignments defining a specific behavior for implementing the non-accomplished assignment. When the selected assignment is selected from the in-depth assignment list 231, the new assignment selection unit 2031 may set the relevant assignment as one of the assignments specifying a behavior for implementing the non-accomplished assignment. When the selected assignment is selected from the additional assignment list 232, the relevant assignment may be set as one of the assignments specifying a behavior for implementing the parent assignment of the non-accomplished assignment. Thus, by clarifying the relationship between the assignments, determination that a certain assignment can be replaced by a different assignment, and the like, is made easier.

In subsequent periods, information indicating the implementation status of the newly set assignment "read a book by the window or outside" with the assignment ID = T01-03, the assignment "have breakfast by the window" with the assignment ID = T01-04, and the assignment "hang out laundry" with the assignment ID = T01-06 are registered in the Implementation status DB 104. The measure implementation supporting apparatus may include an input means (not shown) for accepting the information indicating the implementation status.

After a predetermined period, the assignment IDs = {T01-03, T01-04, T01-06} of the current assignments are input as the information 11 designating the assignment to the implementation status determination unit 101.

The implementation status determination unit 101 calculates an implementation rate during the predetermined period of the designated assignment, for example, based on the information indicating the implementation status of each assignment every day as illustrated in Fig. 17, and determines that the implementation of the assignment is insufficient when the calculated implementation rate is smaller than or equal to a predetermined value. In the case of the example illustrated in Fig. 17, the implementation rate of a predetermined period (e.g., seven days) of the assignment with assignment ID = T01-03 is calculated as 3/7. Furthermore, the implementation rate of a predetermined period (e.g., seven days) of the assignment with assignment ID = T01-04 is calculated as 3/7. Moreover, the implementation rate of a predetermined period (e.g., seven days) of the assignment with assignment ID = T01-06 is calculated as 2/7. Assuming the threshold value is 0.5, the implementation is determined as insufficient for all the assignments. The implementation status determination unit 101 outputs the assignment IDs = {T01-03, T01-04, T01-06} as the information of the assignments the implementation of which is insufficient. Thus, based on the information indicating the implementation status of the designated assignment stored in the implementation status DB 104, the implementation status determination unit 101 may determine that the implementation is insufficient when the percentage or the number of failures to implement the designated assignment is smaller than a predetermined threshold value, and determine that the implementation is sufficient when the percentage or the number of failures to implement the designated assignment is greater than or equal to the predetermined threshold value.

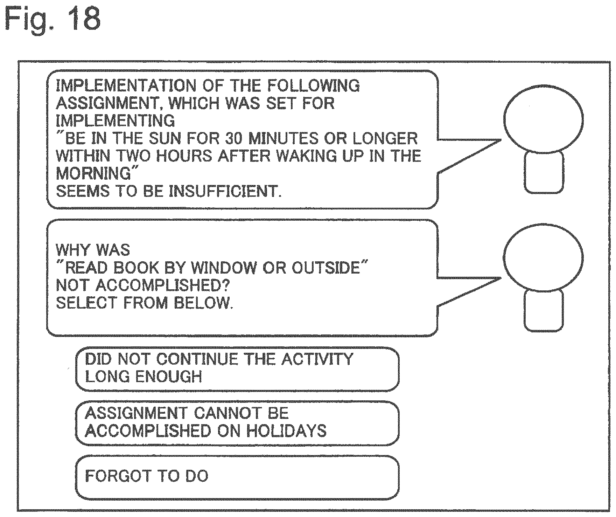

When the implementation status determination unit 101 determines that the implementation is insufficient for any of the assignments, the reason acquisition unit 102 asks the user the reason for failure for each of the assignments (non-accomplished assignments). In the present example, the reason acquisition unit 102 first acquires the reason list associated with the assignment ID = T01-03, which is one of the non-accomplished assignments, from the assignment association table 2053 illustrated in Fig. 13. The reason acquisition unit 102 then asks the user to select a reason for failure among the reasons for failure included in the acquired reason list presented as options (see Fig. 18). Similarly for the other non-accomplished assignments with the assignment IDs = T01-04, T01-06, the reason acquisition unit 102 similarly acquires a reason list from the assignment association table 2053 and asks the user a reason for failure.

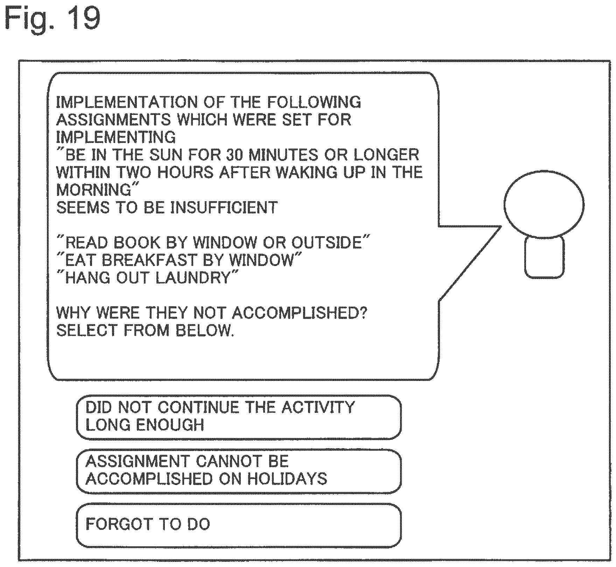

When there is more than one non-accomplished assignment, the reason acquisition unit 102 may ask the user the reason for failure for all the non-accomplished assignments together. For example, the reason acquisition unit 102 acquires the reason list associated with each non-accomplished assignment from the assignment association table 2053 illustrated in Fig. 13. The reason acquisition unit 102 may ask the user to select a relevant reason for failure for the group of non-accomplished assignments from among the reasons of failure included in the union of the acquired reason lists presented as options (see Fig. 19). In this case, the user may select one reason for failure, or may select all the relevant reasons for failure.

The implementation status determination unit 101 may constantly check the implementation status of the highest order assignment (in the case of present example, the assignment with the assignment ID = T01-01). The highest order assignment related to the lifestyle improvement measure may be the one to be accomplished, and the individual lower order assignments specifying a behavior for implementing the highest order assignment do not necessarily need to be accomplished. For example, even if a lower order assignment is given, the user may be asked to input the implementation status of the highest order assignment. The user may be asked to input the implementation status of the lower order assignment as well as the highest order assignment set for the user. In such a case, the implementation status determination unit 101 may output information on whether the implementation of the highest order assignment is insufficient in addition to or in place of the assignment ID of the non-accomplished assignment.

Upon receiving information that the implementation of the highest order assignment is insufficient, for example, the reason acquisition unit 102 may inquire the reason for failure of the highest order assignment. In this case, the reason acquisition unit 102 may also inquire the reason for failure, if known, of the lower order assignment the implementation of which is determined to be insufficient, or the inquiry of the reason for failure of the lower order assignment may be omitted.

In the present example, suppose that the user selects the reason for failure "did not continue the activity long enough" with the reason ID = R01-01 as the reason for failure of the highest order assignment. Upon receiving the selection result, the reason acquisition unit 102 obtains the reason ID = {R01-01} as information 12 indicating the reason for failure of the non-accomplished assignment (in the case of present example, the highest order assignment). When the reason for failure of the lower order assignment is inquired, a pair of an assignment ID and a reason ID of the non-accomplished assignment is further obtained as information 12 indicating the reason for failure.

The reason acquisition unit 102 input the pairs of an assignment ID and a reason ID = {{T01-03, R01-01}, {T01-04, R01-01}, {T01-06, R01-01}} of the non-accomplished assignments to the new assignment selection unit 2031. More than one reason ID may be included. The new assignment selection unit 2031 takes out the pairs of an assignment ID and a reason ID of the non-accomplished assignments one by one, and registers the assignment ID in the in-depth assignment list 231 and the additional assignment list 232, based on the values of the in-depth flag and the addition flag of the reason for failure indicated by the reason ID and the assignment ID of each non-accomplished assignment.

In the present example, the new assignment selection unit 2031 first takes out the pair of an assignment ID and a reason ID {T01-03, R01-01}. The value of the in-depth flag of the reason ID = R01-01 in the reason table 2052 is "TRUE", and thus the assignment ID = {T01-10}, which has the assignment ID = T01-03 of the non-accomplished assignment as the parent ID, is acquired from the assignment association table 2053. However, the assignment IDs with the reason selection field thereof not containing the reason for failure selected by the user are removed from the assignment IDs acquired from the assignment association table 2053. In the case of the present example, the acquired assignment ID = T01-10 is excluded as the reason selection field thereof contains "R01-03", which does not match R01-01 or the reason for failure selected by the user. Therefore, nothing is registered in the in-depth assignment list 231.

Since the value of the additional flag of the reason ID = R01-01 in the reason table 2052 is "TRUE", the new assignment selection unit 2031 acquires the assignment IDs = {T01-02 to TO 1-07} which share the parent ID = T01-01 of the assignment ID = T01-03, which is the non-accomplished assignment, from the assignment association table 2053. However, the currently set assignments and the parent assignments thereof (recursively up to root) are excluded. In the present example, T01-03, T01-04, T01-06 which have the acquired assignment IDs = {T01-02 to T01-07} are currently set assignments, and thus are excluded. The ultimately remaining assignment IDs = {T01-02, T01-05, T01-07} are registered in the additional assignment list 232.

Next, the new assignment selection unit 2031 takes out the next pair of an assignment ID and a reason ID {T01-04, R01-01}. The new assignment selection unit 2031 registers the relevant assignment ID in the in-depth assignment list 231 and the additional assignment list 232 by a method similar to the case of the first assignment ID = T01-03. In the present example, the value of the in-depth flag of the reason ID = R01-01 in the reason table 2052 is "TRUE", and thus the assignment IDs = {T01-11, T01-12} which have the assignment ID = T01-04 of the non-accomplished assignment as the parent ID, are acquired from the assignment association table 2053. The reason selection field of the acquired assignment IDs = T01-11, T01-12 all contain R01-01, which is the reason for failure selected by the user. Therefore, the assignment IDs = {T01-11, T01-12} are registered to the in-depth assignment list 231.

The parent ID of the assignment ID = T01-04 is the same as the parent ID of the assignment ID = T01-03, and thus the assignment IDs to be registered in the additional assignment list 232 are also the same. Therefore, in the case of the non-accomplished assignment having the same parent ID as the already processed non-accomplished assignment, the processing of the registration to the additional assignment list 232 is omitted.

Next, the new assignment selection unit 2031 takes out the final pair of an assignment ID and a reason ID {T01-06, R01-01}. The new assignment selection unit 2031 registers the relevant assignment IDs in the in-depth assignment list 231 and the additional assignment list 232 by a method similar to the case of the first assignment ID = T01-03. In the present example, the value of the in-depth flag of the reason ID = R01-01 in the reason table 2052 is "TRUE", and thus an attempt is made acquire the assignment IDs which have the assignment ID = T01-06 of the non-accomplished assignment as the parent ID from the assignment association table 2053, but the relevant assignment IDs are not found, and hence additional registration to the in-depth assignment list 231 is not carried out.

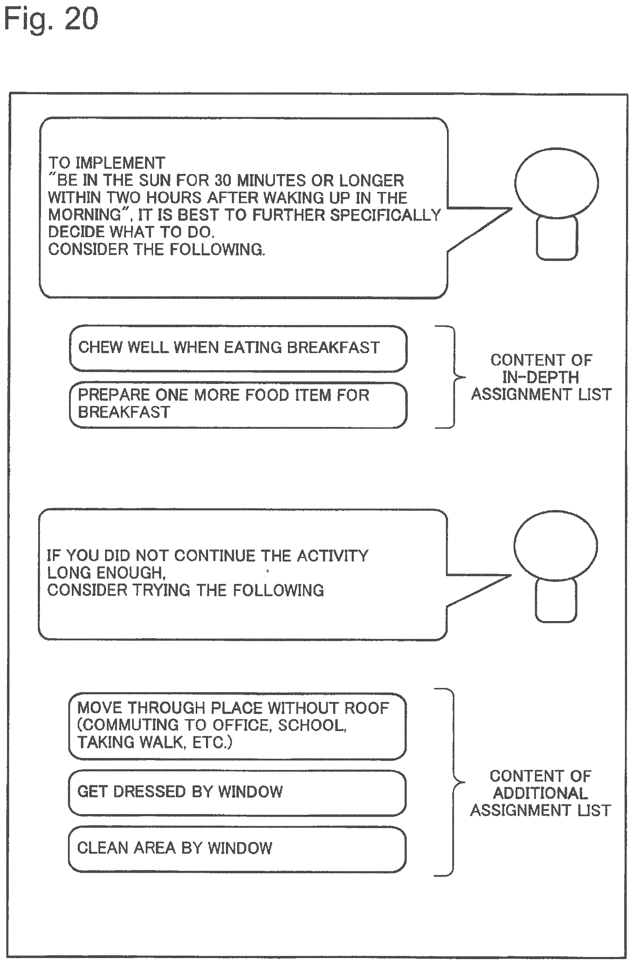

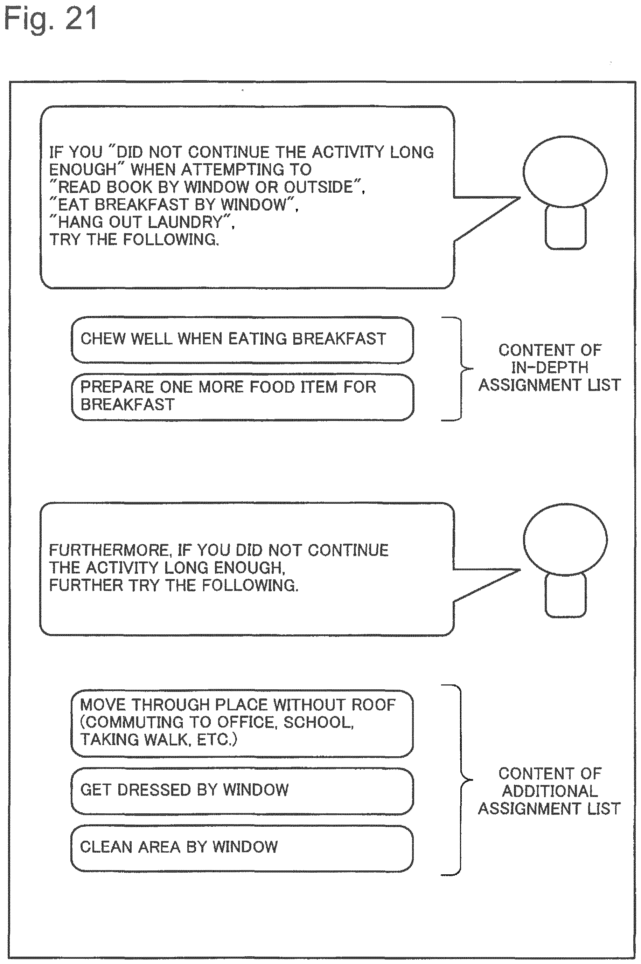

Lastly, the message output unit 2032 presents the created in-depth assignment list 231 and the additional assignment list 232 to the user along with the recommendation message (see Fig. 20). Fig. 20 illustrates an example of using the content of the highest order assignment in the message when presenting the in-depth assignment list 231, but the content and the reason for failure of each non-accomplished assignment can also be used (see Fig. 21).

As described above, according to the present exemplary embodiment, whether it is preferable to further specify the behavior or to add another specific behavior can be set with respect to each reason for failure, so that a finer response can be made for every reason for failure in addition to the effect of the first exemplary embodiment. For example, response in response to the type of second assignment can be carried out such as not making the implementation for the assignment set from the additional assignment list essential.

In each exemplary embodiment described above, an example of presenting a message to encourage continuation of the assignment the implementation of which is determined to be sufficient has been described, but setting of a new assignment may be recommended for the assignment the implementation of which is determined to be sufficient. In the case of an assignment insufficiently implemented, an assignment specifying a behavior that can accomplish the relevant assignment is preferably given as a new assignment since the effect of the assignment is not confirmed, while in the case of an assignment sufficiently implemented, the response may be changed depending on whether the effect has been observed. For example, in the second exemplary embodiment, the reason acquisition unit 102 may ask the user about the presence/absence of effect with respect to the assignment sufficiently implemented to acquire the presence/absence of the effect. According to the presence/absence of the effect acquired by the reason acquisition unit 102, the message output unit 2032 encourages the continuation if the effect is present, and creates a list of assignments involving a specific behavior different from the assignment in question by a method similar to the method for creating the additional assignment list 232 if effect is absent. The message output unit 2032 may present the assignment in the created list with a message recommending a new assignment that replaces the assignment in question (the assignment without effect).

Furthermore, the measure implementation supporting apparatus of the present invention may be incorporated in a CBT-I tool that receives input of data related to sleep such as sleep diary, and analyzes the same to assign an assignment to the user.

In such a case, the implementation status determination unit 101 may analyze the input data and determine the implementation status of the relevant assignment when the assignment related to the lifestyle improvement measure related to the sleep schedule is set.

When, for example, an assignment (a highest order assignment) related to the lifestyle improvement measure "restrict the time to be in bed from ○○ o'clock to ○○ o'clock" is set, the implementation status determination unit 101 may determine that the implementation of the assignment is insufficient when the number of days of going to bed earlier than scheduled is greater than or equal to a predetermined number of times. Whether one is in bed earlier than scheduled can be determined by, for example, measuring a difference of the time one went into bed and the sleep time.

When it is determined that the implementation of the assignment related to the lifestyle improvement measure is insufficient, the reason acquisition unit 102 may ask the user whether it is a voluntary failure (whether the user intentionally went to bed early, etc.), an involuntary failure (became the user was drowsy and could not stay awake, etc.), or an oblivious failure (the user forgot the assignment, lost track of time, etc.).

In such a case, when the reason for failure is a voluntary failure, the new assignment selection unit 1031 and the new assignment selection unit 2031 may, instead of carrying out the selection of a new assignment, cause the message output unit 2031 and the message output unit 2032 to present a message describing the intention of setting the assignment in question, such as it is better to aim for a long-term effect than a short-term effect. When the failure caused by the same reason continues, a new assignment specifying a behavior for implementing the assignment may be selected, and the setting thereof may be recommended by a method similar to the method used in each exemplary embodiment described above. When, on the other hand, the reason for failure is the involuntary failure or the oblivious failure, a new assignment specifically defining a devisal for staying awake or a devisal for not forgetting may be selected and the setting thereof may be recommended by a method similar to the method used in each exemplary embodiment described above. In order to determine whether the failure caused by the same reason is continued, for example, the currently set assignment may be stored as history information with the implementation status of the assignment, reason for failure, presence/absence of effect, and the like for every elapse of a predetermined period.

Further, for example, a case in which an assignment (a highest order assignment) related to the lifestyle improvement measure "restrict the time to be in bed from ○○ o'clock to ○○ o'clock" is set will be described. In this case, the implementation status determination unit 101 may request the new assignment selection unit 1031 and the new assignment selection unit 2031 to select an assignment specifying a behavior to be implemented after getting out of bed as when one cannot fall asleep for a predetermined time or longer a new assignment, regardless of whether the implementation of the assignment is sufficient when in a predetermined state during the predetermined period. This is to prevent a habit of recognizing the bed as a place other than for sleeping. Being in a predetermined state during a predetermined period means, for example, that the number of days in which mid-awakening, early morning awakening, or difficulty initiating sleep occurred for ○○ minutes or longer during the predetermined period is greater than or equal to a predetermined number of times. In this case, the reason for failure is "involuntary failure".

Not limited to the assignment related to the lifestyle improvement measure about the sleep schedule, the implementation status determination unit 101 may analyze the input data related to sleep, and automatically determine whether the implementation of the currently set assignment or the highest order assignment thereof is sufficient.

Furthermore, not limited to the assignment related to the lifestyle improvement measure for the sleep schedule, the reason acquisition unit 102 may ask the user when the reason for failure of the non-accomplished assignment is any one of "involuntary failure", "voluntary failure", or "oblivious failure". The new assignment selection unit 1031 and the new assignment selection unit 2031 may determine whether to explain the intension to the user or to carry out the selection of the new assignment according to such types of reasons for failure. In such a case, the reason table 1052 and the reason table 2052 can be omitted. The reason acquisition unit 102 may ask the user the reason for failure, based on the reason table 1052 and the reason table 2052, and then determine to which one of the types the obtained reason for failure belongs. Such configuration can be realized, for example, by providing a type field in the reason table 1052 and the reason table 2052.

### Examples of involuntary failures include,

- Get drowsy even when trying not to take a nap
- Can stay outside only for 20 minutes even when trying to be in the morning sun for 30 minutes
- Tend to sleep more than the prescribed time on holidays
- Tried not to drink alcohol, but was invited and forced to drink
- Exercised but stopped due to a strong palpitation and breath shortness
- Exercised but could not continue due to pain in a joint
- Beverage frequently consumed turned out to be containing a lot of caffeine
- Planned to have breakfast, but had no appetite at all
Involuntary failures typically occur because although there is motivation the method is not appropriate, the method is difficult, or the method is not known.

### Examples of oblivious failures include,

- Did not notice that the time had come
- Bathed before going to bed by habit
- Walked underground by habit.
Oblivious failures typically occur because one may forget the assignment itself when implementing the assignment, or may not notice that the time for implementation has come.

### Examples of voluntary failures include,

- Smoked a cigarette before going to bed, knowing that it was wrong
- Could not resist the temptation to drink alcohol
- should have exercised relaxation before going to bed but it was too much of a bother
- Watched a favorite TV program although it was before going to bed. Voluntary failures typically occur because one does not feel the significance of implementation, cannot withstand temptation, or thinks of it as too much of a bother and chooses not to implement.

In the case of such classification, it is assumed effective in the case of the involuntary failure to make the behaviors in assignments more specific so that the patient can adopt a method suitable to him/herself. It is also assumed effective in the case of the oblivious failure to make arrangements so as not to forget assignments such as using an alarm or using a note. Moreover, it is assumed effective in the case of the voluntary failure to again carry out education related to the significance of each assignment as the problem cannot be solved simply by devising good assignments.

The user of the present invention may be a patient himself/herself, a doctor who dialogues with the patient, and the like. When the user is a patient, the CBT-I tool functions as a self-check tool. When the user is not a patient, the CBT-I tool functions as a supporting tool for the doctor and the like who dialogues with a patient. When operated by a user who is not a patient, the operator (user) inputs information obtained from the patient to the CBT-I tool, asks the patient the reason for insufficient implementation or proposes a new assignment with reference to a message displayed on the screen of the CBT-I tool.

In each exemplary embodiment described above, an example in which the new assignment selection unit is arranged in the new assignment recommendation unit has been described, but the measure supporting implementation apparatus may be configured to output the information of the selected new assignment in response to a request by the user without making the recommendation on its own. In such a case, a new assignment information output unit (not illustrated) including the above-described new assignment selection unit and an output unit that outputs the information of the new assignment selected by the new assignment selection unit may be arranged in place of the new assignment recommendation unit.

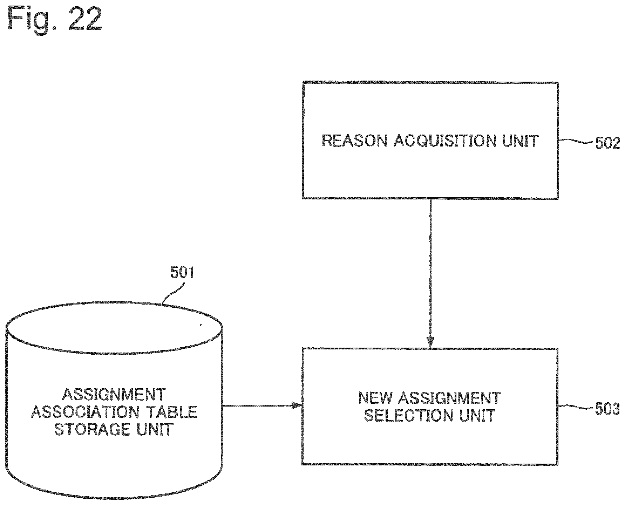

The outline of the present invention will now be described. Fig. 22 is a block diagram illustrating an outline of the present invention. As illustrated in Fig. 22, the measure implementation supporting apparatus of the present invention includes an assignment association table storage unit 501, a reason acquisition unit 502, and a new assignment selection unit 503.

The assignment association table storage unit 501 (e.g., the assignment DB 105, the assignment DB 205) stores an assignment association table in which identification information of an assignment for implementing a predetermined lifestyle improvement measure and identification information of a reason for failure defined in advance with respect to the relevant assignment are at least associated with identification information of another assignment. Here, another assignment is an assignment for implementing the same lifestyle improvement measure, and is an assignment that involves a specific behavior in response to the reason for failure thereof.

When the implementation of the assignment is insufficient, the reason acquisition unit 502 (e.g., the reason acquisition unit 102) allows the user to select the relevant reason for failure from the reasons for failure defined in advance with respect to the designated assignment.

The new assignment selection unit 503 (e.g., the new assignment selection unit 1031, the new assignment selection unit 2031) selects a new assignment for implementing the same lifestyle improvement measure as the designated assignment, based on the identification information of the designated assignment, the identification information of the reason for failure selected by the user, and the assignment association table.

According to such configuration, based on the information of the new assignment selected by the new assignment selection unit 503, the user can specify the behavior for implementing the assignment so that the lifestyle improvement measure can be easily carried out without great trouble whereby the implementation rate and the continuity of the implementation of the assigned lifestyle improvement measure can be increased.

Furthermore, the measure implementation supporting apparatus may include a reason table storage unit (e.g., an assignment DB 105, an assignment DB 205) for storing a reason table in which the identification information of the reason for failure is associated with information related to the reason for failure. The reason acquisition unit may allow the user to select the relevant reason for failure among the reasons for failure, presented as options, defined in advance with respect to the designated assignment, based on the assignment association table and the reason table.

The measure implementation supporting apparatus may include a message output unit (e.g., a message output unit 1032, a message output unit 2032) for outputting a message recommending a new assignment selected by the new assignment selection unit 503.

The reason table storage unit may store a reason table including the reason type information indicating the type of reason for failure as the information related to the reason for failure. In such a case, the types of reason for failure include the involuntary failure, the voluntary failure, and the oblivious failure, and the new assignment selection unit carries out the setting of the new assignment when the type of the reason for failure selected by the user is the involuntary failure or the oblivious failure. The message output unit may output a message explaining the significance of the designated assignment when the type of the reason for failure selected by the user is the voluntary failure.

The reason acquisition unit may cause the user to select the presence/absence of the effect of the designated assignment when the implementation of the designated assignment is sufficient, and the new assignment selection unit may select a new assignment for implementing the same lifestyle improvement measure as the designated assignment when the implementation of the designated assignment is sufficient but the effect is not recognized. The message output unit may output a message recommending that the new assignment selected by the new assignment selection unit be set in place of the designated assignment.

The reason table storage unit may store a reason table including such information that the type of new assignments that can be set when the reason for failure is the relevant one, as the information related to the reason for failure. In such a case, the type of new assignments includes an additional assignment representing an assignment in a parallel relation with the associated assignment, and an in-depth assignment representing an assignment in a subordinate relation with the associated assignment. In the assignment association table, the identification information of the assignment and the identification information of the reason for failure are associated with another assignment that matches the type of new assignments permitted by the reason for failure. The new assignment selection unit may select, as a new assignment, an assignment for implementing the same lifestyle improvement measure as the designated assignment, the assignment being an in-depth assignment with respect to the designated assignment or an additional assignment with respect to the higher order assignment of the designated assignment, based on (A) to (D) below.
(A) Identification information of the designated assignment
(B) Identification information of the reason for failure selected by user
(C) Assignment association table
(D) Type of the second assignments permitted by reason for failure selected by user

The measure implementation supporting apparatus may include an implementation status determination unit (e.g., implementation status determination unit 101) for determining the implementation status of the designated assignment.

The implementation status determination unit may determine the implementation status of the highest order assignment when the designated assignment is not the highest order assignment in the assignment group for implementing the same lifestyle improvement measure.

Furthermore, the measure implementation supporting apparatus is a measure implementation supporting apparatus that supports the implementation of the lifestyle improvement measure assigned to the patient based on the concept of cognitive behavioral therapy for insomnia. The implementation status determination unit may determine the implementation status of the predetermined assignment for implementing the lifestyle improvement measure related to the sleep schedule, based on the data related to the sleep of the patient.

As described above, the present invention has been described with the exemplary embodiments described above as exemplary examples. However, the present invention is not limited to the exemplary embodiments described above. In other words, the present invention can apply various modes that can be recognized by those skilled in the art within the scope of the present invention.

The present application claims priority based on Japanese Patent Application No. 2014-029710 filed on February 19, 2014, the entire disclosure of which is incorporated herein.

### Industrial Applicability

The present invention is not limited to the application for supporting the implementation of the lifestyle improvement measure assigned to the patient based on the concept of the cognitive behavioral therapy for insomnia, and is suitably applicable to applications for supporting the implementation of the predetermined measure in which a specific behavior is not defined.

### Reference signs List

- 100, 200: Measure implementation supporting apparatus
- 101: Implementation status determination unit
- 102: Reason acquisition unit
- 103, 203: New assignment recommendation unit
- 1031, 2031: New assignment selection unit
- 1032, 2032: Message output unit
- 104: Implementation status database (implementation status DB)
- 105, 205: Assignment database (assignment DB)
- 1051: Assignment table
- 1052, 2052: Reason table
- 1053, 2053: Assignment association table
- 501: Assignment association table storage unit
- 502: Reason acquisition unit
- 503: New assignment selection unit

## Claims

1. A measure implementation supporting apparatus comprising:
assignment association table storage means for storing an assignment association table in which identification information of an assignment for implementing a predetermined lifestyle improvement measure and identification information of a reason for failure defined in advance for the assignment are at least associated with identification information of another assignment for implementing the lifestyle improvement measure, the another assignment involving a specific behavior in response to the reason for failure;
reason acquisition means for, when implementation of a designated assignment is insufficient, allowing a user to select a relevant reason for failure from reasons for failure defined in advance with respect to the assignment; and
new assignment selection means for selecting a new assignment for implementing the same lifestyle improvement measure as the designated assignment, based on the identification information of the designated assignment, the identification information of the reason for failure selected by the user, and the assignment association table.

2. The measure implementation supporting apparatus according to claim 1, further comprising
reason table storage means for storing a reason table in which information related to the reason for failure is associated with the identification information of the reason for failure,
wherein the reason acquisition means allows the user to select a relevant reason for failure from reasons for failure, presented as options, defined in advance with respect to the designated assignment based on the assignment association table and the reason table.

3. The measure implementation supporting apparatus according to claim 1 or 2, further comprising
message output means for outputting a message recommending the new assignment selected by the new assignment selection means.

4. The measure implementation supporting apparatus according to claim 3,
wherein the reason table storage means stores a reason table including, as information related to the reason for failure, reason type information indicating a type of the reason for failure,
wherein the type of the reason for failure includes a voluntary failure, an involuntary failure, and an oblivious failure,
wherein the new assignment selection means carries out selection of a new assignment when the type of the reason for failure selected by the user is the involuntary failure or the oblivious failure, and
wherein the message output means outputs the message explaining significance of the designated assignment when the type of the reason for failure selected by the user is the voluntary failure.

5. The measure implementation supporting apparatus according to claim 3 or 4,
wherein the reason acquisition means allows the user to select presence/absence of an effect of the designated assignment when implementation of the designated assignment is sufficient,
wherein the new assignment selection means selects an assignment for implementing the same lifestyle improvement measure as the designated assignment when the implementation of the designated assignment is sufficient but the effect is not recognized, and
wherein the message output means outputs the message recommending that the new assignment selected by the new assignment selection means be set in place of the designated assignment.

6. The measure implementation supporting apparatus according to any one of claims 1 to 5,
wherein the reason table storage means stores a reason table including, as information related to the reason for failure, information indicating a type of a new assignment that can be set when the reason for failure is appropriate,
wherein the type of the new assignment includes an additional assignment representing an assignment in a parallel relation with an associated assignment, and an in-depth assignment representing an assignment in a subordinate relation with an associated assignment,
wherein, in the assignment association table, another assignment matching the type of a new assignment permitted by the reason for failure is associated with the identification information of the assignment and the identification information of the reason for failure, and
wherein the new assignment selection means selects, as the new assignment, an in-depth assignment with respect to the designated assignment or an additional assignment with respect to a higher order assignment of the designated assignment, the in-depth assignment and the additional assignment being an assignment for implementing the same lifestyle improvement measure as the designated assignment, based on the identification information of the designated assignment, the identification information of the reason for failure selected by the user, the assignment association table, and the type of a second assignment permitted by the reason for failure selected by the user.

7. The measure implementation supporting apparatus according to any one of claims 1 to 6, further comprising
implementation status determination means for determining an implementation status of the designated assignment.

8. The measure implementation supporting apparatus according to claim 7,
wherein the implementation status determination means determines the implementation status of the highest order assignment when the designated assignment is not a highest order assignment in a group of assignments for implementing the same lifestyle improvement measure.

9. The measure implementation supporting apparatus according to claim 7 or 8,
wherein the measure implementation supporting apparatus supports implementation of a lifestyle improvement measure assigned to a patient based on a concept of cognitive behavioral therapy for insomnia, and
wherein the implementation status determination means determines an implementation status of a predetermined assignment for implementing a lifestyle improvement measure related to sleep schedule, based on data related to sleep of the patient.

10. A measure implementation supporting method comprising:
storing, in advance, in a predetermined storage means an assignment association table in which identification information of an assignment for implementing a predetermined lifestyle improvement measure and identification information of a reason for failure defined in advance for the assignment are at least associated with identification information of another assignment for implementing the lifestyle improvement measure, the another assignment involving a specific behavior in response to the reason for failure;
when implementation of a designated assignment is insufficient, allowing a user to select a relevant reason for failure from reasons for failure defined in advance with respect to the assignment; and
selecting a new assignment for implementing the same lifestyle improvement measure as the designated assignment, based on the identification information of the designated assignment, the identification information of the reason for failure selected by the user, and the assignment association table.

11. A computer readable recording medium recorded with a measure implementation supporting program for causing a computer accessible to an assignment association table stored in a predetermined storage means, the assignment association table being an assignment association table in which identification information of an assignment for implementing a predetermined lifestyle improvement measure and identification information of a reason for failure defined in advance for the assignment are at least associated with identification information of another assignment for implementing the lifestyle improvement measure, the another assignment involving a specific behavior in response to the reason for failure, to execute:
when implementation of a designated assignment is insufficient, processing of allowing a user to select a relevant reason for failure from reasons for failure defined in advance with respect to the assignment; and
processing of selecting a new assignment for implementing the same lifestyle improvement measure as the designated assignment, based on the identification information of the designated assignment, the identification information of the reason for failure selected by the user, and the assignment association table.
